# EUROPÄISCHE PATENTSCHRIFT

(11) **EP 0 634 176 B1**
(45) Veröffentlichungstag und Bekanntmachung des Hinweises auf die Patenterteilung: **09.02.2000**
(21) Anmeldenummer: 94108973.2
(22) Anmeldetag: 10.06.1994
(51) Int. Cl.: A61L 2/06, F16L 59/16

(54) **Isolation von Rohrleitungen und Armaturen bei einem Dampfsterilisator**
Insulation of pipes and fittings in a steam steriliser
Isolation des conduites et de la robinetterie dans un stérilisateur à vapeur

(30) Priorität: 14.06.1993 DE 4319399
(43) Veröffentlichungstag der Anmeldung: 18.01.1995
(73) Patentinhaber: C. STIEFENHOFER GmbH, D-86971 Peiting (DE)
(72) Erfinder: Kammermeier, Bernhard, Dipl.-Ing., D-82396 Fischen/Ammersee (DE)
(74) Vertreter: Schaumburg, Thoenes, Thurn Patentanwälte

(56) Entgegenhaltungen:
- WO-A-83/03458
- DE-A- 3 819 229
- DE-A- 3 937 023
- DE-B- 1 260 695
- DE-C- 3 929 906
- PATENT ABSTRACTS OF JAPAN vol. 8 no. 276 (M-346) ,18.Dezember 1984 & JP-A-59 145444 (DAIKIN KOGYO KK) 20.August 1984,

## Beschreibung

Die Erfindung betrifft die Isolation von Rohrleitungen und Armaturen bei einem Dampfsterilisator.

Bei Dampfsterilisatoren werden die Rohrleitungen und Armaturen isoliert, um Energieverluste zu vermeiden und auch, um eine Abkühlung sowie evtl. Kondensation des erhitzten Steriliserdampfes außerhalb der Sterilisierkammer zu verhindern.

Bisher werden die Rohrleitungen mit Isolationsmaterial umwickelt. Dieser Vorgang ist aufwendig und erfordert einen erheblichen zeitlichen Arbeitseinsatz. Die Isolation muß an jedes Rohr, an jeden Rohrbogen, an jeden Rohrabzweig, angepaßt und einzeln sorgfältig montiert werden. Armaturen werden üblicherweise gar nicht isoliert, um sie für die Wartung zugänglich zu halten.

Außer den Rohrleitungen und Armaturen müssen die Wände der Sterilisierkammer noch zusätzlich isoliert werden.

Bisher wird die Berohrung in Form eines räumlich uneinheitlichen, dreidimensionalen Gebildes geführt.

Der Erfindung liegt die Aufgabe zugrunde, den Aufwand an Arbeitszeit und Material für die Isolation von Rohrleitungen und Armaturen bei Dampfsterilisatoren zu verringern.
Diese Aufgabe wird erfindungsgemäß durch die im Anspruch 1 angegebenen Merkmale gelöst.

Die Wärmeschutzplatte kann gleichzeitig auch die Wärmedämmung für die betreffende Seitenfläche des Sterilisierdruckbehälters bilden. Für die optimale Umsetzung dieses Prinzips ist es erforderlich, Ventile in Eckbauform einzusetzen.
Dies ist aus 2 Gründen erforderlich:
1. Durch die Eckbauform ist die zweidimensionale Anordnung der Berohrung an der Seitenwand der Sterilisierkammer besonders platzsparend möglich.
2. Durch die Eckbauform ragen die Ventilantriebe im rechten Winkel aus der "Berohrungsebene" heraus, was sich günstig auf die WärmeschutzPlatte auswirkt.

Die Isolation ist so rationell und preiswert herstellbar und kann auch unkompliziert und schnell angebracht und für Wartungszwecke wieder demontiert werden.

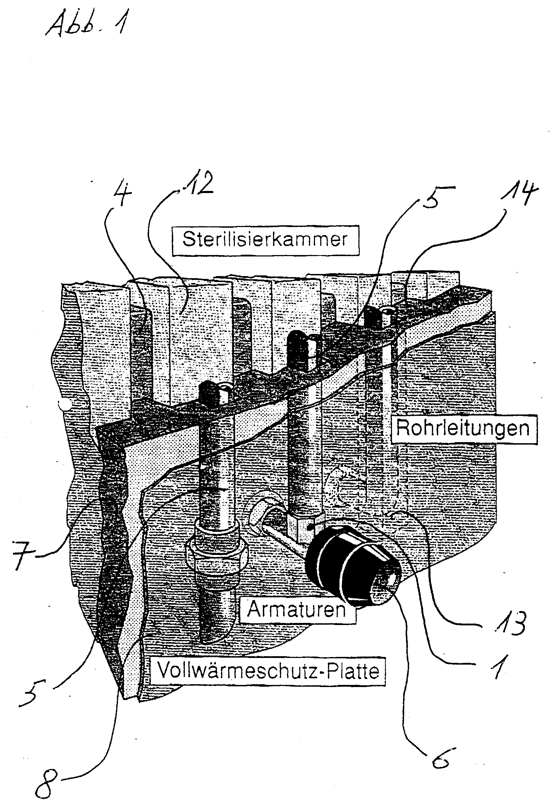

In Abbildung 1 sind die Anordnung der Rohrleitungen, Armaturen und einer Isolationsplatte an der Außenwand einer Sterilisierkammer dargestellt.
In Abb. 2 ist der Einbau eines Eckventils dargestellt.
In Abb. 3 ist zum Vergleich der Einbau eines Schrägsitzventils dargestellt.

Die Außenwand 4 einer Sterilisierkammer eines Dampfsterilisators ist mit Versteifungsprofilen 12 verstärkt. Die Versteifungsprofile 12 sind erforderlich, da in der Sterilisierkammer während des Sterilisationsprozesses Drücke auftreten, die erheblich vom Athmosphärdruck der Umgebung abweichen können. Die Verrohrung 5 für den Dampfsterilisator ist weitgehend in einer Ebene verlegt. Dies ist möglich bei Verwendung von Eckventilen 1 anstelle der sonst üblichen Schrägsitzventile. Eine Isolationsplatte 7 ist u. U. mit Rippen 14 vorgeformt, die in die Zwischenräume der Versteifungsprofile 2 der Außenwand 4 der Sterilisierkammer eingefügt werden können. Die Isolationsplatte 7 hat außerdem Aussparungen 13 eingeformt, in die die Rohrleitungen 5 sowie Armaturteile, wie das Eckventil 1 z. B., passen. Die Isolierplatte wird bei dieser, weitgehend in einer Ebene angeordneten, Verrohrung problemlos über die gesamte Verrohrung und die zu isolierenden Armaturteile aufgesteckt. Sie isoliert damit wirkungsvorll als Vollwärme-schutzplatte sowohl Verrohrung 5, Armaturteile 1 und die Außenwand 4 der Sterilisierkammer eines Dampfsterilisators. Eine Abdeckung 8 ist über der Isolierplatte 7 angebracht. Sie schützt die Oberfläche der Isolierplatte 7 und erhöht auch die Dichtheit der Isolation, da in der Abdeckung 8 die Durchgangsöffnungen zur Umgebung einfacher und genauer eingeformt werden können. Die Abdeckung 8 kann wie die Isolierplatte 6 aus flexiblem Werkstoff hergestellt sein.

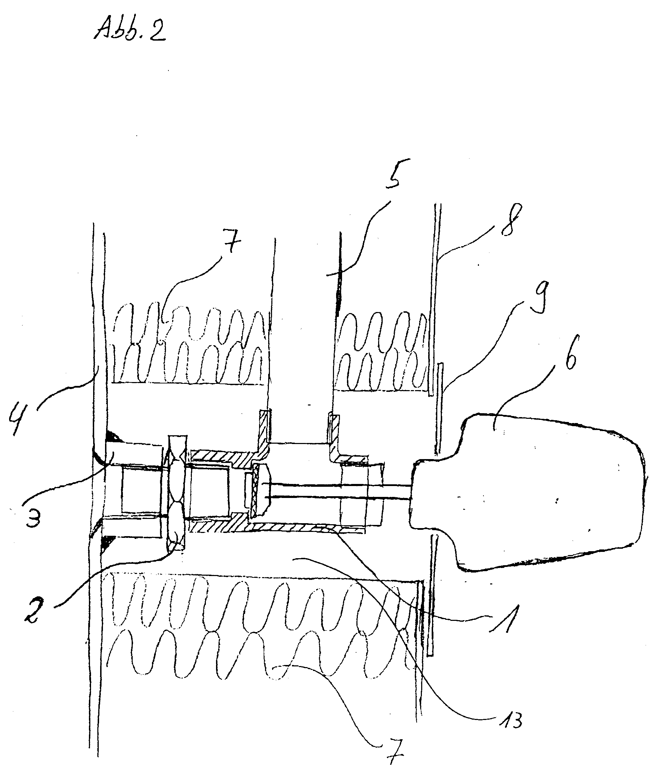

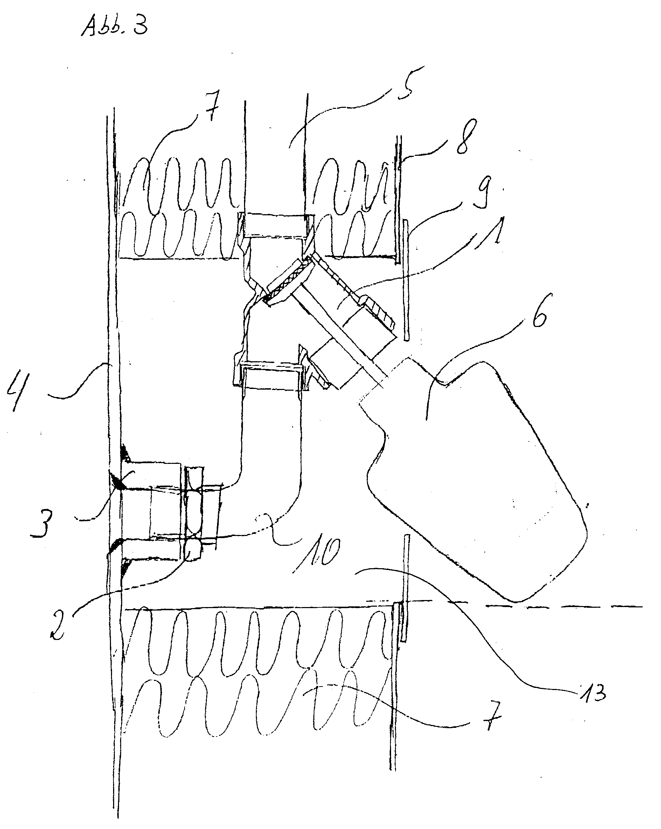

Die Abbildung 2 zeigt die spezielle Anordnung der Eckventile 1. Durch geeignete Verbindungsstücke, z. B. Doppelnippel 2, werden sie direkt mit den Stutzen 3 an der Seitenwand 4 der Sterilisierkammer verschraubt. Dadurch, daß beim Eckventil die Anschlüsse im 90° Winkel zueinander stehen, baut sich die Berohrung 5 zwangläufig in einer parallelen Ebene zur Seitenwand 4 der Sterilisierkammer auf.
Die Ventilantriebe 6 (Pneumatik- bzw. Elektromagnetantriebe) müssen aus der Wärmeschutzplatte herausragen, damit sie nicht unzulässig heiß werden. Die Wärmeschutzplatte 7 muß im Bereich des Ventils ausgespart sein, damit sie über den Ventilantrieb drübergeschoben und wieder abgenommen werden kann.
An der Außenseite der Isolierplatte kann zu deren Schutz eine Abdeckung 8 angebracht sein, die fest mit der Isolierplatte verbunden ist, z. B. durch Klebung. Die Aussparungen im Bereich der Ventile können mit einer abnehmbaren Blechrosette 9 verschlossen sein. Anstelle der Blechrosette können auch flexible Bürstenleisten angeordnet sein. Diese können mit der Abdeckung 8 auch fest verbunden sein, da sie beim Abnehmen der Wärmeschutzplatte durch ihre Flexibilität dem Ventilantrieb ausweichen. Zum Vergleich ist in Abb. 3 dargestellt, wie ungünstig sich die Einbauverhältnisse ergeben würden, wenn anstelle der Eckventile die bisher üblichen Schrägsitzventile 11 eingesetzt würden. Die Berohrung 5 baut sich nicht zwangsläufig in einer parallelen Ebene zur Seitenwand 4 der Sterilisierkammer auf. Es müssen zusätzliche Rohrbogen 10 eingesetzt werden, wodurch Bauraum verlorengeht. Der Ventilantrieb 6 ragt nicht im 90° Winkel aus der Berohrungsebene. Um die Abnehmbarkeit der Wärmeschutzplatte zu gewährleisten, muß der Ausschnitt in dieser wesentlich größer ausgeführt werden als bei der Berohrung mit Eckventilen. Dadurch ist die Wärmedämmwirkung wesentlich schlechter.

Bei Einsatz von Durchgangsventilen würde der Antrieb zwar auch im 90° Winkel aus der Berohrung abstehen, jedoch haben diese Ventile schlechte Durchflußwerte.

## Patentansprüche

1. Isolation von Rohrleitungen (5) und Armaturen (1) an einem Dampfsterilisator, **gekennzeichnet** durch eine Isolierplatte (7), welche die weitgehend in einer Ebene verlegte und Eckventile (1), Eintritts- und Austrittsanschlüsse bilden einen Winkel von 90° zueinander, einschließende Verrohrung zur Wärmeisolierung abdeckt und die Aussparungen (13) hat, die an die Konturen der Rohrleitungen (5) und der Armaturen (1) zur Aufnahme derselben angepaßt sind.

2. Isolation von Rohrleitungen und Armaturen bei einem Dampfsterilisator nach Anspruch 1, dadurch **gekennzeichnet**, daß die Isolierplatte zur Wärmeisolation der Verrohrung und der Armaturen gleichzeitig die Wärmedämmung der angrenzenden Außenwand der Sterilisierkammer darstellt.

## Claims

1. Insulation of pipelines (5) and fittings (1) on a steam steriliser, characterised by an insulating plate (7) which for thermal insulation covers the piping which is laid substantially in one plane and includes corner valves (1), inlet and outlet connections form a 90° angle to one another, and which has openings (13) adapted to the contours of the pipelines (5) and the fittings (1) for the purpose of accommodating the same.

2. Insulation of pipelines and fittings in a steam steriliser according to claim 1, characterised in that the insulating plate for the thermal insulation of the piping and fittings simultaneously forms the thermal insulation of the adjoining outer wall of the sterilising chamber.

## Revendications

1. Isolation de conduites en tuyau rigide (5) et d'armatures (1) dans un stérilisateur à vapeur, caractérisée par une plaque isolante (7), laquelle recouvre pour l'isolation thermique les conduites situées dans une large mesure dans un plan et incluant des clapets d'équerre (1) dont les raccords d'entrée et de sortie forment entre eux un angle de 90°, et comporte des évidements (13) qui sont adaptés au contour des conduites (5) et des armatures (1) pour le logement de celles-ci.

2. Isolation de conduites et d'armatures dans un stérilisateur à vapeur selon la revendication 1, caractérisée en ce que la plaque isolante pour l'isolation thermique des conduites et des armatures réalise en même temps l'isolation thermique de la paroi extérieure adjacente de la chambre de stérilisation.
